# EUROPÄISCHE PATENTSCHRIFT

(11) **EP 0 559 689 B1**
(45) Veröffentlichungstag und Bekanntmachung des Hinweises auf die Patenterteilung: **24.06.1998**
(21) Anmeldenummer: 91920320.8
(22) Anmeldetag: 18.11.1991
(51) Int. Cl.: A61L 31/00, A61L 27/00

(54) **HOCHFESTE UND ABBAUBARE WERKSTOFFE UND FORMKÖRPER FÜR DIE IMPLANTATION IN DEN MENSCHLICHEN UND TIERISCHEN ORGANISMUS**
HIGH-STRENGTH BIODEGRADABLE MATERIALS AND MOULDED ARTICLES FOR IMPLANTATION IN THE HUMAN AND ANIMAL BODY
MATERIAUX ET CORPS MOULES TRES RESISTANTS ET DEGRADABLES IMPLANTABLES DANS DES ORGANISMES HUMAINS ET ANIMAUX

(30) Priorität: 26.11.1990 DE 4037516
(43) Veröffentlichungstag der Anmeldung: 15.09.1993
(73) Patentinhaber: MERCK PATENT GmbH, 64271 Darmstadt (DE)
(72) Erfinder: RITTER, Wolfgang, D-5657 Haan (DE)
(86) Internationale Anmeldenummer: EP9102169
(87) Internationale Veröffentlichungsnummer: WO9209313

(56) Entgegenhaltungen:
- EP-A- 0 274 319

## Beschreibung

Die Erfindung betrifft neue hochfeste Werkstoffe auf Kunststoffbasis und daraus gefertigte Formteile, die im menschlichen und tierischen Organismus abbaubar und resorbierbar sind.

### Zum Stand der Technik

In der klinischen Operationstechnik ist der Ersatz von metallischen Implantaten durch resorbierbare organische Polymere, welche die Zweitoperation zur Entfernung der Implantate erübrigen, erwünscht. Im praktischen Einsatz sind heute resorbierbare Fadenmaterialien und resorbierbare Stifte zur Fixierung von Knochenfragmenten bekannt. Diese bisher im Einsatz befindlichen Materialien sind aus Polymilchsäure, Polyglykolsäure, Polydioxanon bzw. aus deren Copolymeren hergestellt.

Die technische Nutzung solcher Materialien in der Form resorbierbarer Platten, Schrauben, Nägel und dergleichen ist bisher ausgeblieben. Verantwortlich sind dafür unter anderem die folgenden Mängel der bis heute bekannten Materialien: Die Polymerfestigkeit liegt zu niedrig, so daß beispielsweise beim Einsatz dieser Materialien als Schrauben die Köpfe abplatzen. Es besteht eine starke Abhängigkeit der Endfestigkeit vom eingestellten Molgewicht. Die Verarbeitung der Materialien ist extrem aufwendig.

Die DE-A1 32 29 635 beschreibt chirurgische Bindemittelsysteme zum Verkleben von körpereigenem Hartgewebe gewünschtenfalls mit Kunststoff und/oder Metall. Vorgesehen ist hier eine resorbierbare (Meth)-acrylatkomponente, die bei Raumtemperatur flüssig bis fest ist und aus (Meth)acrylsäureestern mit (Meth)-acrylatresten an Oligoesterketten aus Hydroxycarbonsäuren besteht, wobei diese Oligoester aus niederen Monohydroxymonocarbonsäuren gebildet sind. Als Polymerisations-auslösender Starter sind hier zwingend Organoborverbindungen vorgeschrieben, die sich durch eine besonders hohe Aktivität bezüglich der Polymerisationsauslösung auszeichnen. Dieser auf eigene Arbeiten der Anmelderin zurückgehenden Druckschrift lag die Erkenntnis zugrunde, daß für die notwendige Aushärtung zu hochfesten Fugenverbindungen im Bereich chirurgischer Klebstoffe die Kombination der Organoborverbindungen als Startersystem mit den insbesondere polyfunktionellen (Meth)acrylsäureestern besonders zweckmässig sein kann.

Angesprochen wird in diesem älteren Schutzrecht auch das Bedürfnis, resorbierbare Stützmaterialien in der operativen Technik einzusetzen, um hierdurch auf die sonst erforderliche zweite Operation verzichten zu können. Dementsprechend beschreibt die genannte Druckschrift neben der Verwendung des reaktiven Zweikomponentensystems dessen Verwendung zur in-situ-Herstellung von resorbierbaren Formteilen bei der Verklebung von körpereigenem Hartgewebe, gegebenenfalls zusammen mit Kunststoff und/oder Metall. Zwingender Bestandteil des so ausgebildeten Formteiles und damit in den lebenden Organismus zu implantierender Inhaltsstoff des Formteils sind die als Starter eingesetzten Organoborverbindungen. Bis zum heutigen Tage haben sich Materialien dieser Art im praktischen Einsatz nicht durchgesetzt.

### Zum Gegenstand der Erfindung

Die Lehre der vorliegenden Erfindung stellt ein neues Material für den angegebenen Einsatzbereich zur Verfügung, das sich durch extrem hohe und dazu einstellbare Materialfestigkeit auszeichnet, auf die an sich bekannte Klasse von Oligohydroxycarbonsäure-poly(meth)acrylaten zurückgreift, dabei aber auf den Einsatz von Startersystemen auf Basis Bor-organischer Verbindungen verzichtet.

Gegenstand der Erfindung sind dementsprechend im menschlichen und tierischen Organismus abbau- und resorbierbare hochfeste Werkstoffe und entsprechende vorgebildete Formkörper, wie Implantate, auf Basis gehärteter (Meth)acrylsäureester von unter Mitverwendung mehrwertiger Alkohole oder mehrwertiger Carbonsäuren (hier mit nachfolgenden Umsetzung mit mehrwertigen Alkoholen bzw. deren Derivaten) mehrfunktionell hydroxyl-terminierten Oligomeren Hydroxycarbonsäuren (im folgenden als mehrfunktionelle (Meth)acrylsäureester bezeichnet) mit bis zu 10 C-Atomen. Hierbei erfaßt der Begriff der (Meth)acrylsäureester sowohl die entsprechenden Ester der Acrylsäure als der Methacrylsäure, wobei auch Mischungen dieser beiden ethylenisch ungesättigten Säurekomponenten im gleichen Molekül vorliegen können.

Die erfindungsgemäße Lehre ist dadurch gekennzeichnet, daß die Werkstoffe bzw. die daraus gefertigten Formkörper für die Implantation in den lebenden Organismus durch Strahlenhärtung und/oder durch eine sonstige, jedoch Bor-freie radikalische Polymerisation der mehrfunktionellen (Meth)acrylsäureester dreidimensional vernetzt sind und dabei eine Zugfestigkeit von wenigstens 10 N/mm² aufweisen.

Bevorzugt ist, daß die Kunststoff-Formkörper eine Zugfestigkeit von wenigstens 15 N/mm² und insbesondere entsprechende Werte von wenigstens 30 N/mm² aufweisen. Typische Werkstoffe bzw. Formteile im Sinne der Erfindung zeigen Zugfestigkeitswerte im Bereich von etwa 40 bis 60 N/mm². Die Zugfestigkeitswerte liegen damit deutlich oberhalb der Festigkeitswerte typischer gesunder menschlicher Knochen. Eine zur Bestimmung der Zugfestigkeitswerte geeignete Verfahrensmethodik wird im Nachfolgenden noch angegeben.

In einer weiteren Ausgestaltung des erfindungsgemäßen Handelns wird es darüberhinaus möglich, Werkstoffe bzw. Formkörper der geschilderten Art zur Verfügung zu stellen, die von physiologisch bedenklichen Reaktionshilfsmitteln und/oder sonstigen Zusatzstoffen aus der Herstellung der Werkstoffe (im folgenden als Wirkstoffverunreinigung bezeichnet) soweit nach Art und Menge frei sind, daß physiologisch unerwünschte Sekundäreffekte im Rahmen des Körpergeschehens beim Abbau und bei der Resorption dieser Implantate nicht zu befürchten sind. Die Erfindung berücksichtigt in dieser Ausführungsform insbesondere die in vergleichsweise geringen Konzentrationen im Fertigprodukt vorliegenden Kleinkomponenten, die durch Implantation des Kunststoffkörpers in den lebenden Organismus eingetragen werden.

Ein wichtiger Kern der Erfindung, auf den nachfolgend noch ausführlich eingegangen wird, liegt dabei in konkret gewählten Parametern für die Herstellung der neuen Werkstoffe bzw. Formkörper. Die Erfindung sieht hier insbesondere eine Abstimmung zwischen den Inhibitoren der mehrfunktionellen (Meth)acrylsäureester einerseits und den Bedingungen zur Auslösung der Vernetzung dieser mehrfunktionellen (Meth)acrylsäureester vor. Diese Abstimmung erfolgt dabei jeweils nach Art und Menge der eingesetzten Hilfsstoffe dergestalt, daß die notwendigerweise im ausreagierten Feststoffmaterial zurückbleibenden und dort eingebundenen Hilfsstoffe aus der Fertigung beim nachfolgenden Abbau des Polymermoleküls im Zuge der körpereigenen Resorption bedenkenlos freigesetzt und vom Körper aufgenommen werden können.

### Einzelheiten zur erfindungsgemäßen Lehre

Die erfindungsgemäße Lehre stellt es in ihrem Kern darauf ab, Reaktivkomponenten der geschilderten Art zu entwickeln und daraus Werkstoffe bzw. Formteile zu bilden, die jetzt bezüglich ihrer - in den aufeinanderfolgenden Fertigungsstadien - zwingend erforderlichen Hilfsstoffe in Richtung auf Körperverträglichkeit optimiert sind. Hierzu gilt dann das folgende:

Es ist chemisches Grundwissen, daß bei Herstellung, Transport und/oder Lagerung radikalisch reaktiver Systeme die Mitverwendung von Radikalinhibitoren zum sicheren Ausschluß einer unerwünscht frühzeitigen Abreaktion des Systems notwendig ist. Die Praxis kennt hier zahlreiche Verbindungen bzw. Systeme. In Betracht kommen beispielsweise Hydride wie Lithiumaluminiumhydrid, Calciumhydrid oder Natriumborhydrid. Weitere bekannte Beispiele für diesen Zweck sind Phenole, Phenolderivate, Hydrochinon und Hydrochinonderivate oder insbesondere Phenothiazin.

Solche Radikalinhibitoren werden dabei nicht erst für die sichere Lagerung der Systeme bis zum Zeitpunkt der Applikation benötigt, schon bei der Herstellung der radikalisch reaktiven Verbindungen - beispielsweise der mehrfunktionellen (Meth)acrylsäureester - wird der Einsatz solcher Inhibitoren benötigt. Es läßt sich dementsprechend zwischen den sogenannten "Herstellungsinhibitoren" und den "Applikationsinhibitoren" unterscheiden, wobei diese beiden Typen der Inhibitoren nach Art und/oder Menge gleich oder ungleich sein können. Im ersten Fall ist der Applikationsinhibitor im allgemeinen der bereits bei der Herstellung eingesetzte Herstellungsinhibitor. Gerade die hohe Vergelungstendenz mehrfunktioneller (Meth)acrylsäureester der hier betroffenen Art fordert jedoch bei der Herstellung den Einsatz stark wirksamer Herstellungsinhibitoren, deren Mitverwendung bei der Applikation und letztlich damit als Bestandteil des in den lebenden Körper zu implantierenden Formstücks unerwünscht sein kann.

Nach einem wesentlichen Element der Erfindung sind die jetzt beschriebenen Werkstoffe bzw. Implantate frei von - nach Art und/oder Menge - unerwünschten Inhibitoren aus der Herstellung, Lagerung und/oder Verarbeitung der mehrfunktionellen (Meth)acrylsäureester.

Ein besonders wichtiges Beispiel für einen geeigneten Inhibitor im Sinne des erfindungsgemäßen Handelns sind Tocopherolverbindungen und hier insbesondere das Alpha-tocopherol und damit das Vitamin E.

Der Einsatz von physiologisch verträglichen Tocopherolverbindungen und insbesondere Vitamin E als Applikationsinhibitor, gegebenenfalls aber auch schon als Herstellungsinhibitor im Zusammenhang mit mehrfunktionellen (Meth)acrylsäureverbindungen der auch erfindungsgemäß betroffenen Art ist im einzelnen Gegenstand der älteren Anmeldung P 39 39 161.2 (D 8930) der Anmelderin. Die Offenbarung dieses älteren Schutzrechtes wird hiermit ausdrücklich auch zum Gegenstand der Erfindungsoffenbarung gemacht.

Zur Vervollständigung der Erfindungsoffenbarung wird im nachfolgenden kurz auf den Inhalt dieser älteren Anmeldung eingegangen. Das Vitamin E kann alleiniger Applikationsinhibitor zur Stabilisierung der mehrfunktionellen (Meth)acrylsäureester eingesetzt sein, wobei seine Verwendung in Mengen von etwa 200 bis 10000 ppm, üblicherweise im Bereich von etwa 300 bis 4000 ppm - jeweils bezogen auf das Gewicht des radikalisch reaktiven Stoffgemisches - bevorzugt ist. In einer wichtigen Ausführungsform ist das Vitamin E nicht nur als Applikationsinhibitor vorgesehen. Hier wird Vitamin E auch schon als Herstellungsinhibitor bei der Synthese der letztlich mit dem lebenden Körper in Kontakt tretenden Massen eingesetzt. Auf diese Weise wird in einer wichtigen Ausführungsform die Übertragung unerwünschter Inhibitorbestandteile in die Implantate und damit in den lebenden Organismus verhindert. Wichtig ist das insbesondere im Zusammenhang mit der Herstellung der mehrfunktionellen (Meth)acrylsäureester, die bekanntlich keiner Reinigung durch Destillation unterworfen werden können.

Gemäß einer anderen Ausführungsform der Erfindung ist allerdings auch ein Inhibitoraustausch möglich, wie er insbesondere in der älteren Patentanmeldung P 39 39 162 (D 8929) der Anmelderin beschrieben ist. Nach der Lehre dieses älteren Schutzrechtes werden als Herstellungsinhibitoren entsprechende Verbindungen des Phenoltyps mit zur Salzbildung befähigten Hydroxylgruppen eingesetzt, die dann nach der Herstellung der Reaktivkomponenten durch Abreaktion mit festen basischen Komponenten, insbesondere festen Oxiden und/oder Hydroxiden der Erdalkalimetalle, gebunden und aus dem Reaktionsgemisch entfernt werden. Der auf diese Weise abgetrennte Herstellungsinhibitor wird durch Vitamin E als Applikationsinhibitor ersetzt.

(Meth)acrylsäureester mehrfunktioneller Alkohole sind schon immer als eine extrem vergelungs-gefährdete Stoffklasse erkannt und beschrieben. Der Stand der Technik sieht für ihre Herstellung häufig zur Minderung der Gefahr einer vorzeitigen Ab- bzw. Anreaktion den Einsatz von Lösungsmitteln vor, um durch den Verdünnungseffekt die Gefahr der frühzeitigen Vergelung zu mindern. Die vergleichsweise leichtflüchtigen Lösungsmittel werden nach den Angaben des Standes der Technik dann destillativ abgezogen, wobei häufig durch längere Behandlung im Hochvakuum versucht wird, auch letzte Lösungsmittelreste abzutrennen. Es hat sich jedoch gezeigt, daß in aller Regel die wirklich vollständige Abtrennung der Lösungsmittel schwierig ist, so daß hier eine zusätzliche Quelle einer unnötigen Belastung des lebenden Organismus bei der Implantation von Formteilen aus solchen Reaktivsystemen vorliegt. In einer bevorzugten Ausführungsform der Erfindung soll auch eine solche Belastung zuverlässig ausgeschaltet werden. Dementsprechend wird hier im Rahmen der Herstellung der erfindungsgemäßen Werkstoffe bzw. Formteile auf die Mitverwendung von insbesondere physiologisch bedenklichen Lösungsmitteln von vorneherein verzichtet. Verwiesen wird in diesem Zusammenhang auf die Offenbarung der auf die Anmelderin zurückgehenden Druckschriften gemäß DE-A1 38 43 854, 38 43 938, 38 43 930 und 38 43 843.

Mehrfunktionelle Reaktivkomponenten für den Aufbau der erfindungsgemäßen Werkstoffe bzw. Formteile sind die Oligomerkomponenten, wie sie in den vorveröffentlichten DE-A1 32 04 504 und 32 29 635 der Anmelderin beschrieben sind. Es handelt sich hierbei um Systeme, die als mehrfunktionelle (Meth)acrylsäureester entsprechende Verbindungen enthalten, die bei Raumtemperatur flüssig bis fest sind und im Molekül wenigstens 2 (Meth)acrylatreste an aus Hydroxycarbonsäuren gebildeten Oligoesterketten enthalten. Geeignet können insbesondere Verbindungen mit 2 bis 4 (Meth)acrylatresten an den Oligomersegmenten sein. In den für die praktische Ausbildung der erfindungsgemäßen Hilfsstoffe besonders wichtigen Verbindungstypen liegen 2 und/oder 3 (Meth)acrylatreste am Oligomermolekül vor, wobei insbesondere im Fall der 2fach substituierten Oligomersegemente die Alpha-, Omega-Stellung der (Meth)acrylatreste bevorzugt sein kann.

Die Oligomersegmente weisen das Strukturmerkmal 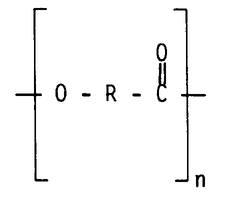 auf. Sie sind durch Oligomerisierung geeigneter Hydroxycarbonsäuren bzw. Hydroxycarbonsäuregemische der allgemeinen Formel 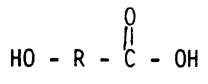 zugänglich. In einer bevorzugten Ausführungsform der Erfindung sind die Reste - R - und n derart ausgewählt bzw. aufeinander abgestimmt, daß das mittlere Molekulargewicht der Polyester-Oligomereinheit im Bereich von etwa 100 bis 600 liegt. Besonders bevorzugte Werte für das mittlere Molekulargewicht liegen in diesem Bereich oberhalb 120, vorzugsweise bei oder oberhalb 150 - 200 und insbesondere im Bereich von etwa 300 bis 500.

Das Polyester-Oligomersegment wird in einer bevorzugten Ausführungsform aus Monohydroxymonocarbonsäuren gebildet, die eine C-Zahl bis etwa 10 im Molekül aufweisen. Niedere Hydroxycarbonsäuren mit 2 bis 6 C-Atomen sind besonders wichtig. Besonders geeignete Hydroxycarbonsäuren zur Ausbildung dieses Mittelstücks der mehrfunktionellen (Meth)acrylatverbindungen sind Glykolsäure, die verschiedenen isomeren Milchsäuren, die gegebenenfalls isomeren Alpha- oder Beta-Hydroxypropionsäuren, die gegebenenfalls isomeren Alpha-, Beta- oder Gamma-Hydroxybuttersäuren und/oder deren Gemische. Es können dabei sowohl bestimmte Isomere der genannten Säuren als auch beliebige Gemische zum Einsatz kommen. Die wichtigsten Vertreter der hier genannten Grundkörper sind die Glykolsäure und die Milchsäure.

Die Polyesteroligomeren sind dabei zweckmäßigerweise unter Mitverwendung von mehrfunktionellen Reaktanten hergestellt. Durch diese Coreaktanten erfolgt die Regelung des mittleren Molekulargewichts im Polyesteroligomeren. Zum anderen ist durch Auswahl der funktionellen Gruppen der mitverwendeten Coreaktanten die Möglichkeit gegeben, an den Polyesteroligomeren einheitlich endständige Hydroxylgruppen oder endständige Carboxylgruppen auszubilden.

Der Einsatz von polyfunktionellen Alkoholen als Coreaktanten ist bevorzugt. Geeignet sind insbesondere 2- bis 4-wertige Alkohole. Besondere Bedeutung kann hier niederen polyfunktionellen Alkoholen zukommen, wobei besonders herauszustellen sind das Ethylenglykol, die Propandiole, hier insbesondere das 1,2-Propandiol und das Glycerin.

In allen Fällen entstehen modifizierte Oligoester, die in an sich bekannter Weise einfach zu den mehrfunktionellen (Meth)acrylsäureestern umgesetzt werden können. Liegen endständige Hydroxylgruppen am Oligoester vor, so werden die (Meth)acrylsäuregruppen durch Veresterung bzw. Umesterung mit Acrylsäure oder Acrylsäureestern und/oder insbesondere entsprechenden Methacrylsäureverbindungen eingeführt. Aber auch bei der Mitverwendung von polyfunktionellen Carbonsäuren als reaktive Coreaktanten unter primärer Ausbildung von Oligoestern mit endständigen Carboxylgruppen gelingt die Bildung von geeigneten mehrfunktionellen (Meth)acrylsäureestern. Hier werden die zunächst gebildeten Carboxyl-terminierten Oligomeren mit mehrwertigen Alkoholen bzw. deren Derivaten - beispielsweise mit Glycidylestern der Acrylsäure bzw. der Methacrylsäure - umgesetzt. Auf diese Weise kann letztlich auch hier der erfindungsgemäß geforderte Typ der mehrfunktionellen (Meth)acrylsäureester-Oligomeren gewonnen werden, wie es beispielsweise in der DE-A1 32 29 635 beschrieben ist.

Für die erfindungsgemäße Zielsetzung, die hochfesten Werkstoffe bzw. in bestimmter Raumform ausgebildeten Implantate unter zuverlässiger Kontrolle der im Rahmen des Herstellungsverfahrens anfallenden Wirkstoffverunreinigungen gewinnen zu können, ist in einer bevorzugten Ausführungsform der Erfindung der physikalische Zustand der mehrfunktionellen (Meth)acrylsäureester bei Raumtemperatur oder bei mäßig erhöhten Temperaturen wichtig. Die Erfindung bevorzugt hier die Verarbeitung von fließfähigen oder wenigstens noch pastös verstreichbaren reaktiven Materialien, um ohne Mitverwendung von Lösungsmitteln eine vor der Aushärtung beabsichtigte formgestaltende Verarbeitung zu erleichtern oder erst zu ermöglichen. Wichtig kann diese physikalische Beschaffenheit des noch nicht gehärteten reaktiven Materials insbesondere auch im Zusammenhang mit der Einarbeitung von Füllstoffen in die Formkörper und/oder für deren schichtenartigen Aufbau sein, worauf im nachfolgenden noch eingegangen wird. Es kann dabei bevorzugt sein, daß die mehrfunktionellen (Meth)acrylatverbindungen bei Raumtemperatur eine Viskosität im Bereich von etwa 500 bis 70000 mPas, vorzugsweise im Bereich von etwa 3000 bis 50000 mPas besitzen. Der Einsatz von Gemischen einer Mehrzahl solcher Reaktivkomponenten auf Oligomerbasis unterschiedlichen Aufbaus und damit unterschiedlicher physikalischer Eigenschaften ist in die Lehre des erfindungsgemäßen Handelns eingeschlossen. Gerade in diesem Zusammenhang kann schließlich eine Ausführungsform von Bedeutung sein, die die Mitverwendung sogenannter reaktiver Verdünner vorsieht, die insbesondere der Klasse 1-funktioneller (Meth)acrylsäureester zuzuordnen sind. Coreaktanten dieser Art werden beim dreidimensionalen Vernetzungsprozeß zur Ausbildung der hochfesten Werkstoffe bzw. Formkörper in die Polymerstruktur eingebunden.

Ein hinreichendes rheologisches Verhalten der mehrfunktionellen (Meth)acrylsäureester bei Raumtemperatur oder nur mäßig erhöhten Temperaturen - beispielsweise bis zu etwa 50 bis 60 °C - kann aber auch unter Berücksichtigung der nachfolgenden Überlegungen wesentlich sein. Wesentliche Verfahrensstufe bei der Herstellung der neuen dreidimensional vernetzten Werkstoffe bzw. Formkörper ist die Härtung der Masse durch insbesondere radikalische Abreaktion der ethylenisch ungesättigten (Meth)acrylsäurereste am Oligomerenmolekül unter gleichzeitiger dreidimensionaler Vernetzung. Diese Reaktionsauslösung kann in verschiedener Weise bewerkstelligt werden. Beherrschender Gedanke muß im Sinne der erfindungsgemäßen Zielsetzung auch hier sein, die in diesem Zusammenhang gegebenenfalls einzuarbeitenden Reaktionshilfsstoffe (Initiatoren, Startersysteme und dergleichen) nach Art und Menge so zu beschränken, daß die beim Abbau frei werdenden Reststoffe keine physiologischen Probleme auslösen. Wichtig ist dafür insbesondere die Möglichkeit der homogenen Einarbeitung gegebenenfalls mitzuverwendender Hilfsstoffe in den bzw. die zu härtenden Reaktanten. Diese homogene Einmischung wird durch die erfindungsgemäß bevorzugte Rheologie des nichtgehärteten Materials begünstigt und gegebenenfalls überhaupt erst ermöglicht.

Zur Auslösung der radikalischen Härtung kommen insbesondere 2 an sich bekannte Reaktionsmechanismen in Betracht, die Strahlungshärtung und der Einsatz von Radikale bildenden Startersystemen.

Reaktivsysteme der erfindungsgemäßen Art sind strahlenhärtend auch schon bei Einsatz niederer Arbeitstemperaturen. Insbesondere sind sie auch für eine von Photo-Initiatorenfreie Reaktionsauslösung geeignet. So können durch Photopolymerisation insbesondere bei Einsatz von UV-Licht in situ Licht-gehärtete Formkörper hergestellt werden, die aufgrund des polyfunktionellen Monomercharakters ein praktisch unendliches Molgewicht aufweisen und sehr hohe Festigkeitswerte besitzen.

Aufgrund der einfachen Licht-initiierten Polymerisation können leicht Formkörper beliebiger Art hergestellt werden. Die UV-Lichthärtung kann dabei derart stufenweise durchgeführt werden, daß in einer ersten Verfahrensstufe das Material vorgehärtet und formstabilisiert wird, während in einer nachfolgenden Bestrahlungsstufe dann die volle Aushärtung des Materials erreicht wird. Durch Kühlung - beispielsweise durch Wasserkühlung - kann sichergestellt werden, daß festgesetzte Maximaltemperaturen im Formkörper nicht überschritten werden. Es kann dabei notwendig sein, durch geeignete Maßnahmen einen direkten Kontakt des härtenden Formkörpers mit dem Kühlwasser auszuschließen.

Gewünschtenfalls können andere reaktionsauslösende Strahlungstypen, z.B. Laserstrahlen, Röntgenstrahlen oder Gamma-Strahlen, eingesetzt werden. Die Mitverwendung von an sich bekannten Initiatoren ist möglich, verwiesen wird in diesem Zusammenhang beispielsweise auf die druckschriftlichen Angaben in Enzyl. Polym. Sci. and Eng. (2. Auflage) Bd. 11, 187 - 207 und einschlägige Handelsprodukte, beispielsweise der Firmen Merck, Darmstadt (DE) und Ciba-Geigy, Schweiz.

Möglich ist erfindungsgemäß insbesondere aber auch die Härtung des formgestalteten Reaktivmaterials in an sich bekannter Weise durch chemisch initiierte radikalische Polymerisation. Für den Einsatz hier bekannter Hilfsmittel ergibt sich die nachfolgende Erleichterung bei der Auswahl der Reaktionshilfsmittel: Der Stand der Technik kennt eine Vielzahl von Redoxsystemen auf Basis peroxidischer Verbindungen, die in Kombination mit Reduktionsmitteln und/oder Metallverbindungen solcher Metalle eingesetzt werden, die in mehreren Wertigkeitsstufen auftreten können. Verwiesen wird in diesem Zusammenhang beispielsweise auf die zusammenfassende Darstellung in PROGRESS IN POLYMER SCIENCE, Vol. 8, Pergamon Press, Oxford New York, 1982, Seiten 61 bis 131 und die umfangreiche darin zitierte Primärliteratur.

Bei der Auswahl geeigneter Redoxsysteme, die insbesondere nach Art und Menge der im gehärteten Material Zurückbleibenden Reststoffe keine wesentlichen physiologischen Bedenken auslösen, kommt dem Handeln im Rahmen der Erfindung der folgende Tatbestand besonders zugute: Physiologisch vergleichsweise unbedenkliche Reaktantensysteme sind nach dem Stand der Technik insbesondere beschrieben im Zusammenhang mit wäßrigen Polymerisationssystemen. Das erfindungsgemäß zu vernetzende Material ist an sich ein wasserfreies System. Aufgrund seiner Oligomerenstruktur, die auf niedere Hydroxycarbonsäuren zurückgeht, sind jedoch die Löslichkeitsverhältnisse auch im Rahmen der Erfindung für solche Komponenten häufig sichergestellt, wie sie sonst in den wäßrigen Systemen zum Einsatz kommen. Es wird damit möglich, eine wirklich homogene Verteilung physiologisch weitgehend unbedenklicher Aktivatorsysteme auch in der wasserfreien Reaktionsphase der mehrfunktionellen (Meth)acrylsäureester zu erreichen, um damit dann bei vorgegebenen Temperaturen die Vernetzung zu bewirken.

Aus der großen Klasse geeigneter Komponenten für solche Redoxsysteme seien hier lediglich beispielhaft genannt: Peroxidverbindungen wie Persäuren, Diacylperoxide, Hydroperoxide und dergleichen, wobei physiologisch verträglichen Säuren, beispielsweise sogenannten Genußsäuren, als Peroxid bildender Bestandteil besondere Bedeutung zukommen kann. Aus der großen Klasse der Aktivatoren bzw. Reduktionsmittel bieten sich Verbindungen an wie Ascorbinsäure, Sorbose, Fructose, Dextrose oder andere Zucker, bei denen es sich durchweg um physiologisch unbedenkliche Komponenten handelt.

Zur Stimulierung bzw. Aktivierung der Redoxreaktion geeignete Metallverbindungen leiten sich insbesondere vom Eisen ab, das in Form 2-und/oder 3-wertigen Eisens in an sich bekannter Weise den Redoxsystemen zugegeben werden kann. Die homogene Einmischung des Eisens gelingt beispielsweise besonders sicher mit den entsprechenden Salzen der Glykolsäure und/oder der Milchsäure. Die Erfindung ermöglicht die Herstellung hochfester Werkstoffe bzw. Formkörper im Sinne der eingangs gegebenen Definition der Mindestwerte für die Primär-Zugfestigkeit. Zur Ermittlung dieser Werte gelten dabei die Standardbedingungen die im Beispielsteil einleitend ausführlich angegeben sind.

In einer besonderen Ausführungsform der Erfindung sind die neuen Werkstoffe und/oder Formkörper durch einen Gehalt an Füllstoffen gekennzeichnet. Diese Füllstoffe liegen vorzugsweise in diskreter Feststoffphase vor und können dabei sowohl anorganischer wie organischer Natur sein. Die bevorzugten Vertreter dieser Füllstoffklassen sind ihrerseits körperresorbierbar oder aber wenigstens körperverträglich. Beispiele anorganischer Füllstoffe dieser Art sind körperverträgliche Keramikwerkstoffe, insbesondere sogenannte bioaktive Keramikwerkstoffe in Pulver- und/oder in Granulatform. Bekannte Beispiele hierfür sind etwa Tricalciumphosphat und/oder Hydroxylappatit in ungesinterter oder gesinterter Form. Verwiesen wird in diesem Zusammenhang beispielsweise auf die DE-A1 38 25 211 und 38 26 915, in denen verbesserte, überwiegend körperresorbierbare Knochenwachse bzw. neue Werkstoffe für den Knochenersatz und Knochen- bzw. Prothesenverbund beschrieben sind.

Eine andere Klasse sind Füllstoffe mit Faserstruktur. Durch die Armierung der Formteile gemäß der Erfindung mit faserartigen Füllstoffen bzw. aus Fasern gefertigten Füllstoffmaterialien kann es gelingen, die geforderten Stoffeigenschaften zusätzlich positiv zu beeinflussen.

So sind in einer ersten Ausführungsform Werkstoffe bzw. Formteile der erfindungsgemäßen Art zusätzlich mit Stapelfasern armiert bzw. ausgerüstet. Die Stapelfasern werden dabei in einer möglichen Ausführungsform in das noch fließfähige ungehärtete mehrfunktionelle (Meth)acrylsäureestermaterial eingearbeitet und dann beim nachfolgenden Härtungsprozeß fest eingebunden. In einer besonders interessanten Ausführungsform wird der Einbau von Faserbündeln, insbesondere von entsprechenden Materialien mit Fadenstruktur im Formteil bzw. Werkstoff vorgesehen. So ist es beispielsweise möglich, stiftartige Materialien zentral um solche Fäden bzw. Fadenbündel auszubilden. Hier kann es vorgesehen sein, die Füllstoff-Fasern bzw. Fadenmaterialien in vorgespanntem Zustand in das dreidimensionale Netzwerk einzubinden, um die hochfesten Eigenschaften solcher vorgespannten Fäden unmittelbar auf das Formteil im Sinne der Erfindung zu übertragen.

In anderen Ausführungsformen der Erfindung werden neben oder anstelle der bisher dargestellten Faserausrüstung Faden-basierte Materialien wie Netze, Gewebe, Gewirke und dergleichen in das auszuhärtende Material eingearbeitet und in diesem Zustand in das dreidimensionale Netzwerk eingebunden. So kann insbesondere eine Umhüllung eines Werkstoffstabes im Sinne der Erfindung in seinen Außenbereichen mit einem entsprechenden flächenförmigen Fadengebilde vorgesehen sein oder aber es wird ein entsprechendes flächenförmiges Material spiralförmig aufgewickelt, mit dem fließfähigen mehrfunktionellen (Meth)acrylsäureestermaterial durchdringend getränkt und zum hochfesten Formkörper ausgehärtet. Wie bereits angegeben, sind die Füllstoffe und damit insbesondere auch die hier angegebenen Fasern vorzugsweise ihrerseits aus körperresorbierbarem Material gebildet. In diesem Zusammenhang bieten sich insbesondere Fasern bzw. Fäden oder daraus gewonnene weiterführende Materialien auf Basis von Hochpolymeren der Glykolsäure und/oder der Milchsäure an. Fadenförmiges hoch-zugfestes Polymilchsäurematerial ist bekanntlich in der operativen Technik als Nahtmaterial bekannt und in breitem Umfange eingeführt. Geeignet sind aber auch Fäden auf Polypeptid-Basis. Zur Verbesserung des Haftverbundes kann eine Vorbehandlung der Faseroberfläche zweckmäßig sein.

Neben oder auch ohne solche zusätzlichen Hilfsmittel kann erfindungsgemäß eine andere Modifikation der neuen hochfesten Werkstoffe bzw. Formkörper vorgesehen sein. Hierbei handelt es sich um die nachfolgenden Elemente:

Ein bestimmter Formkörper kann nicht nur aus einem bestimmten einheitlichen dreidimensional vernetzbaren Oligomermaterial hergestellt werden - sei es in Form eines bestimmt ausgewählten mehrfunktionellen (Meth)acrylsäureesters oder einer bevorzugt homogenen Mischung mehrerer Ester dieser Art - es ist auch ein bevorzugt schichtartiger Aufbau der Formkörper aus entsprechenden Materialien unterschiedlicher Beschaffenheit möglich. So können Formkörper mit solcher Schichtstruktur beispielsweise Materialschichten einer vergleichsweise schnelleren Körperresorbierbarkeit mit wenigstens einer Materialschicht mit verringerter Hydrophylie und/oder erhöhter Hydrolysebeständigkeit verbinden. Hierbei kann es bevorzugt sein, die Materialschicht(en) der vergleichsweise schnelleren Körperresorbierbarkeit in den Innenbereich des Formkörpers zu legen und diesen Kern mit einer oder mehreren Material schichten erhöhter Hydrolysebeständigkeit abzudecken. Dabei kann ein solcher Kern aus einer oder mehreren Materialschicht(en) mit erhöhter Abbaugeschwindigkeit von einer oder mehreren Materialschicht(en) erhöhter Festigkeit gegen Durchtritt der wäßrigen Körperflüssigkeit wenigstens anteilsweise nach Außen abgedeckt sein.

Die Besonderheit einer solchen Ausgestaltung des Formkörpers leuchtet ein: Bei der Implantation des Formkörpers beispielsweise als Stift in den Kern eines gebrochenen Röhrenknochens wird zunächst für einen verlängerten Zeitraum eine hinreichende Funktionsfähigkeit des Stiftes unter Belastung gefordert. Diese Forderung besteht wenigstens so lange, bis durch Regenerierung des Knochenwachstums die Bruchstelle ihre eigene Belastungsfähigkeit gewonnen hat. Ist dieser Zustand erreicht, kann ein vergleichsweise rascherer Abbau des implantierten Stiftmaterials hingenommen oder sogar wünschenswert sein. Durch die hier skizzierte Möglichkeit der schichtförmigen Ausgestaltung solcher Implantate gelingt eine optimale Anpassung der Gesamteigenschaften des Implantats nicht nur im Zeitraum unmittelbar nach der Implantation, sondern auch im weiteren Verlauf des Heilungs- und Resorptionsprozesses.

Die potentielle Hydrolyseanfälligkeit der dreidimensional vernetzten Werkstoffe bzw. Formkörpermaterialien und der damit verbundene Abfall in den Festigkeitseigenschaften kann es insgesamt wünschenswert machen, insbesondere im Außenbereich eine oder mehrere Lagen von Materialschichten vorzusehen, die erhöhte Festigkeit gegen den Feuchtigkeitsdurchtritt zeigen. Gleichwohl sollen in bevorzugter Ausführungsform auch diese Materialschichten letztlich abbaubar sein. Es hat sich gezeigt, daß hier wieder insbesondere die Milchsäure-basierten Polymermaterialien mit ihrer verringerten Hydrophylie geeignete Hilfsmittel im Sinne der Erfindung sein können. So kann beispielsweise ein vorgebildetes dreidimensionales Werkstück im Sinne der Erfindung in eine Schmelze einer Polymilchsäure eingetaucht werden, wodurch Deckschichten vorbestimmbarer Dicke aus Polymilchsäure aufziehen. Gewünschtenfalls kann auf eine solche Deckschicht nochmals dreidimensionales (Meth)acrylsäureestermaterial im Sinne der Erfindung aufgetragen werden. Hierdurch kann eine unerwünschte Verletzung der Deckschicht erhöhter Wasserfestigkeit bei der Implantation sicher vermieden werden. Geeignet sind auch Deckschichten oder Zwischenschichten auf Basis der nächst höheren Hydroxycarbonsäuren, insbesondere Polyhydroxybutyrat oder Poly-(Hydroxybutyrat-co-hydroxyvalerat).

Die Erfindung kann schließlich davon Gebrauch machen, daß durch Auswahl der die oligomeren Molekülkerne bildenden Bestandteile Einfluß auf die Hydrolysebeständigkeit des vernetzten (Meth)acrylsäureestermaterials genommen werden kann. Vertreter der Milchsäure sind bekanntlich deutlich hydrolysebeständiger als vergleichbare Vertreter der Glykolsäure. Ebenso ist beispielsweise 1,2-Propandiol oleophiler als das Methylgruppen-freie Ethylenglykol. In der zuvor angedeuteten Weise sieht dementsprechend die Erfindung beispielsweise vor, den Kern des Formteils aus einem vergleichsweise rasch abbaubaren Material auf Basis Oligoglykolsäure/Ethylenglykol aufzubauen, während die geforderte Lebensdauer der gewünschten Festigkeitswerte durch äußere Bereiche des Werkstücks auf Basis Milchsäureoligoester gewünschtenfalls unter Mitverwendung von mehrwertigen Alkoholen erhöhter Oleophilie zur Regelung des Molgewichtes ausgestaltet sind.

In einer besonderen Ausgestaltung sieht die Erfindung vor, wenigstens anteilsweise im Formkörper, insbesondere im Formkörperquerschnitt, medizinische Wirkstoffe mitzuverwenden. Gebrauch gemacht wird hier von der an sich bekannten Depotwirkung von Oligomeren der Milchsäure und/oder Glykolsäure für die Einlagerung und zeitgesteuerte Freigabe solcher medizinischer Wirkstoffe. Als Beispiele solcher medizinischer Wirkstoffe sind zu nennen: Mittel mit desinfizierender und/oder antibakterieller Wirkung, wobei hier insbesondere Antibiotika und hier wiederum insbesondere Breitbandantibiotika in Betracht kommen. Andere Beispiele für solche medizinischen Wirkstoffe sind Antiallergika und/oder Mittel zur Stimulierung des Gewebe- bzw. Knochenwachstums. Diese hier lediglich beispielhaft aufgeführten Verbindungsklassen können je nach Anforderungsprofil nahezu beliebig erweitert werden.

Die Implantate der Erfindung können beliebige Raumform aufweisen. Geeignete Raumformen sind beispielsweise Stäbe, Platten, Schienen, Stifte, Schrauben, gitterförmige Elemente und dergleichen. Die Erfindung umfaßt dabei sowohl die serienmäßige Herstellung entsprechender standardisierter Materialien für die operative Praxis als die individuelle Herstellung dem Einzelfall angepaßter Werkstücke insbesondere die in-situ-Herstellung entsprechender Formkörper.

### Beispiele

Die Ermittlung physikalischer Festigkeitswerte, insbesondere der Zugfestigkeit, der Bruchdehnung und des E-Moduls erfolgt an Testkörpern die gemäß den nachfolgenden Angaben hergestellt werden.

Das Einsatzmaterial auf Basis des reaktiven mehrfunktionellen (Meth)acrylsäureesters wird mittels einer Aluminiumform zu einem spatelartigen Festkörper definierter Abmessungen ("Testknochen") vergoßen und in dieser Form ausgehärtet.

Im einzelnen gilt:

Der Testknochen ist als stabförmiger Körper mit rechteckigem Querschnitt ausgebildet, dessen schmaler Mittelteil sich beidseitig an den Formkörperenden verbreitert.

### Daten zur Abmessung des Testknochens:

- Gesamtlänge:: 74 mm
- Dicke:: 1,9 mm
- Länge des mittigen schmalen Teiles:: 27 mm
- Breite des mittigen schmalen Teiles:: 4 mm
- Breite der beiden Endstücke:: 12 mm

Die Aluminiumform besteht aus 2 aneinanderschraubbaren Teilen um den gehärteten Testknochen leichter aus der Form entnehmen zu können. Beim Eingießen der zu härtenden Masse in die Form ist folgendes zu beachten:

Das Monomer muß blasenfrei sein. Beim Eingießen selber dürfen an den Kanten (besonders im schmalen Bereich des Knochens) keine Blasen entstehen. Daher empfiehlt es sich, daß man zuerst mit einer Spritze mit Nadel die Kanten mit Monomer bedeckt und erst dann die Form endgültig auffüllt. Die freiliegende Oberfläche wird dann mit einem Objektträger abgezogen, um so eine glatte Oberfläche zu erhalten.

### Härtung

Zur Härtung wird folgende Lichtquelle eingesetzt:
UV ASpot 400/T
400 W 200/230 V
Dr. Hönle GmbH

Die UV-Birne hat eine Leistung von 400 W, die Entfernung zum belichteten Objekt beträgt 22 cm. Die Intensität des UV-Strahlers bestimmt u.a. die Härtungszeit.

Folgende UVA-Strahlungsleistungen wurden ermittelt:
- 1. In der Kammermitte:: 31,2 mW/cm²
- 2. An den Kammerrändern:: ca. 25,1 mW/cm²

### Festigkeitswerte

Die gehärteten Knochen werden an einer Zerreißmaschine der Fa. Zwick bei Zerreißgeschwindigkeit von 5 mm/min zerrissen. Die Zugfestigkeit wird als Funktion der Härtungszeit gemessen:

### Zugversuche an Testknochen aus gehärtetem Oligo(glycolsäure-Ethylenglycol 4: 1) bismethacrylat

| Probenlänge 27 mm, Breite 4 mm, Dicke 1,9 mm | | | | |
|---|---|---|---|---|
| Beispiel | Härtungsdauer UV 400 W | Zugfestigkeit N/mm² | Bruchdehnung % | E-Modul N/mm² |
| | | (jeweils Mittelwert aus 3 Versuchen) | | |
| 1 | 3 h | 59,1 | 14,9 | 430 |
| 2 | 8 h | 84,8 | 18,7 | 568 |
| 3 | 12 h | 77,7 | 16,1 | 572 |

Es zeigt sich, daß bei ca. 8 h Härtungszeit ein Maximum der Zugfestigkeit liegt. Auch der E-Modul erreicht sein Maximum nach dieser Zeit.

### UV-Härtung des mit Photoinitiator versetzten Monomers

Um die Härtungsdauer zu verkürzen, wurde das Monomer mit zwei verschiedenen Photoinitiatoren versetzt und gehärtet. Folgende Photoinitiatoren kamen zum Einsatz:
1. Irgacure 651/Fa.Merck: 1 Gew.-% gelöst im Monomeren
2. 4-(2-Acryloyloxyethoxy)-phenyl-(2-hydroxy-2-propyl)-keton/Fa. Merck: 1 Gew.-% bzw. 3 Gew.-% gelöst im Monomeren

### Zugversuch an Testknochen aus gehärtetem Oligo(glycolsäure-Ethylenglycol 4: 1) bismethacrylat mit dem Photoinitiator Irgacure 651

| Beispiel | Initiator Konzentration/% | Härtungsdauer UV 400 W | Zugscherfestigkeit N/mm² | E-Modul N/mm² |
|---|---|---|---|---|
| | | | Mittelwert aus 3 Messungen | |
| 4 | 1 | 2 h | 36,2 | 301 |
| 5 | 1 | 4 h | 40,9 | 340 |

### Zugversuch an Testknochen aus gehärtetem Oligo(glycolsäure-Ethylenglycol 4 : 1)bismethacrylat mit dem Photoinitiator 4-(2-Acryloyloxyethoxy)-phenyl-(2-Hydroxy-2-propyl)-keton

| Beispiel | Initiator Konzentration/% | Härtungsdauer UV 400 W | Zugscherfestigkeit N/mm² | E-Modul N/mm² |
|---|---|---|---|---|
| | | | Mittelwert aus 3 Messungen | |
| 6 | 1 | 4 h | 43,7 | 312 |
| 7 | 1 | 8 h | 60,8 | 329 |
| 8 | 3 | 4 h | 72,8 | 479 |

### Faserverstärkung

In gleicher Weise - wie bisher beschrieben - wurden in das Monomer 10 Gew.-% von ca. 4 mm langen Kurzfasern, geschnitten aus Polyglactin 910-Fasern (Sutupak) der Fa. Ethicon/2000 Norderstedt, eingebettet und die Mischung gehärtet. Folgende Ergebnisse wurden erzielt.

| Beispiel | Härtungszeit | Zahl der Fasern | Zugfestigkeit N/nm² | Bruchdehnung/% |
|---|---|---|---|---|
| 9 | 3 h | Wirrvlies | 44,08 | 14,7 |
| 10 | 6 h | Wirrvlies | 45,35 | 12,1 |

Als mehrfunktionelle (Meth)acrylatverbindung wird das in der älteren Patentanmeldung P 39 39 161.2 bereits beschriebene Oligoglycolsäure (Pn = 4)bismethacrylat eingesetzt, dessen Herstellung nachfolgend nochmals beschrieben ist.

### Herstellung des Oligoglycolsäure-bismethacrylats:

Eine mit Vitamin E stabilisierte Reaktivkomponente auf Basis OligoglykolsaureBismethacrylat wird in den folgenden Verfahrensstufen hergestellt:

Zunächst wird aus Glykolsäure und Ethylenglykol im Molverhältnis 4 : 1 ein Hydroxylgruppen-terminiertes Oligomeres hergestellt. Dann wird handelsübliche Methacrylsäure unter Zusatz von Phenothiazin als Stabilisatorkomponente im Wasserstrahlvakuum destillativ von ihrem Inhibitorgehalt befreit. Die als Destillat übergegangene Methacrylsäure wird mit Vitamin E stabilisiert. Schließlich wird das Oligoglykolsäure-Vorkondensat mit der Methacrylsäure unter Zusatz von p-Toluolsulfonsäure als Katalysator und Zugabe einer weiteren Vitamin E-Menge im lösungsmittelfreien System verestert. Das Fortschreiten der Veresterungsreaktion wird kontrolliert. Erforderlichenfalls werden geringe zusätzliche Mengen an Methacrylsäure nachdosiert.

Das angefallene Reaktionsprodukt wird schließlich auf dem Wege der Trockenneutralisation mit Calciumhydroxid säurefrei gestellt und über einen Druckfilter von dem festen Neutralisationsmittel befreit.

Die eingesetzten Verfahrensschritte werden im nachfolgenden im einzelnen geschildert. Dabei wird mit einer Mehrzahl von Reaktionsansätzen gefahren. Das Alterungsverhalten der unter Luftausschluß gelagerten Oligoglykolsäure-Vorkondensate wird über den Zeitraum eines Jahres bestimmt. Dabei ist Konstanz der Produkteigenschaften festzustellen.

Im einzelnen gilt das folgende:

### 1.1 Herstellung des Oligomeren Glykolsäure/Ethylenglykol 4 : 1

In den 25-Liter-Versuchsreaktor wurden 16,72 kg Glykolsäure und 3,41 kg Ethylenglykol vorgelegt. Nach der Inertisierung wurde der Kristallbrei unter Stickstoff-Strom geschmolzen und dann weiter bis maximal 145 bis 150 °C (Sumpftemperatur) aufgeheizt. Nachdem die Reaktion durch Abdestillation von Wasser eingesetzt hatte, wurde sie 11 Stunden lang weitergeführt, bis sich kein Reaktionswasser mehr abspaltete (Brüdentemperaturabfall auf 70 bis 73 °C bei einem Reaktionsumsatz von 70 %). Von der abdestillierten wäßrigen Lösung wurde zur Quantifizierung die Menge des Destillats, die Säurezahl (Gehalt an Glykolsäure) und der Wassergehalt nach Karl-Fischer bestimmt. Um die Reaktion vollständig durchzuführen, wurde bei 150 °C vorsichtig auf 400 Torr evakuiert und innerhalb von 2 Stunden der Druck weiter bis auf 10 Torr reduziert und 1 Stunde gehalten, damit das restliche Reaktionswasser zur Quantifizierung abgezogen wurde.

Das Kondensat wurde in einer Kühlfalle (gekühlt mit Trockeneis und Ethanol) aufgefangen und im Anschluß ebenfalls quantifiziert. Nach der gesamten Reaktionszeit wird auf 100 °C abgekühlt, das Vakuum mit Stickstoff aufgehoben und das Produkt noch heiß abgefüllt. Das Produkt wurde nicht weiter gereinigt und direkt zur Herstellung von Oligoglykolsäurebismethacrylat eingesetzt.
Ausbeute: 97,7 %

### Analysenergebnisse vom Oligomeren

| Bezeichnung | direkt nach Herstellung | 1 Monat alt | 1 Jahr alt |
|---|---|---|---|
| Ansatzgröße | 20 kg | 4,5 kg | 2 kg |
| Konsistenz | pastös | pastös | pastös |
| Viskosität/b. RT (Epprecht-Viskosimeter MK4) | 12500 mPas | 13000 mPas | 12800 mPas |

| Molgewicht: | | | |
|---|---|---|---|
| Mn* | 438 | 455 | 454 |
| Mw | 515 | 533 | 530 |
| %-freie Glykolsäure | 2,1/2,2 % | 1,4 % | 1,9 % |
| %-freies Ethylenglykol | 0,2 % | 0,2 % | 0,2 % |
| Verseifungszahl | 765,4 | 754,4 | 754,0 |

| Verhalten in Wasser | | | |
|---|---|---|---|
| pH-Wert nach 2 min. | 3,8 | 3,8 | 3,8 |
| pH-Wert nach 60 min. | 3,4 | 3,4 | 3,4 |
| Peroxid-Gehalt | negativ | | |

| | | | |
|---|---|---|---|
| * Bestimmung des Molgewichtes als GPC-Analyse. Da die Eichung mit Polyethylenglykol als Standard durchgeführt wurde, erklärt sich die Differenz zwischen Mn theor. und Mn lt. Molgewichtsbestimmung durch die Eichmethode. | | | |

### 1.2 Oligoglykolsäure-bismethacrylat

Handelsübliche Methacrylsäure (Fa. Roehm) wird nach der folgenden Vorschrift neu mit Vitamin E inhibiert:

In einer Vakuumdestillationsapparatur werden 15 mol (= 1291,35 g) Methacrylsäure (kp. 163 °C mit 3,87 g (= 3000 ppm) Phenothiazin (als Stabilisator) versetzt und unter starkem Luftstrom im Wasserstrahlvakuum überdestilliert. In die Vorlage werden 100 ppm Vitamin E (Covitol F-1000-2, 67%ig, Henkel KGaA) (= 139 mg/l) vorgelegt und unter Rühren die Methacrylsäure überdestilliert. Die Destillation ist beendet, wenn von der Methacrylsäure 932 g überdestilliert sind.

### 1.3 Reaktionsverlauf

In einem Dreihalskolben mit Rührer und Destillationsbrücke wurden 294 g Oligoglykolsäure, 206,4 g Methacrylsäure sowie 17,5 g p-Toluolsulfonsäure eingewogen und mit 0,86 g Vitamin E (Alpha-Tocopherol) inhibiert. Während der gesamten Reaktion wurde Luft mit einer Geschwindigkeit von mindestens 40 l/h eingeleitet.

Durch Abtrennung des entstandenen Wassers bei einer Maximaltemperatur von 105 °C wurde die Veresterung durchgeführt bis die Menge an abgetrenntem Wasser über 35,78 g (mehr als 97 % Umsatz) liegt.

Die Destillationsvorlage wurde während der gesamten Reaktionsführung mit Trockeneis/Ethanolgemisch gekühlt. Bei einer maximalen Sumpftemperatur von 105 °C und bei 500 mbar betrug die Veresterungszeit zwischen 12 und 14,5 Stunden bei einem Gesamtumsatz von 97 bis 98,5 %. Von der abdestillierten wäßrigen Lösung (aus Kühlfalle und Vorlage) wurde zur Quantifizierung alle 1,5 Stunden die Menge des Destillats, die Säurezahl (Gehalt an Methacrylsäure) und der Wassergehalt nach Karl-Fischer bestimmt.

Über eine Differenzberechnung wird der Wassergehalt und die überdestillierte Menge an Methacrylsäure berechnet, wobei nach jeder Bestimmung überprüft wurde, ob noch genügend Methacrylsäure für die Reaktion zur Verfügung stand. Meistens mußte nach ca. 7,5 bis 8 Stunden noch zusätzlich 0,1 mol Methacrylsäure nachdosiert werden.

Nach beendeter Reaktion (Umsatz über 97 %) wurde das Produkt zur Reinigung abgefüllt.

### 1.4 Aufarbeitung des Reaktionsproduktes

Das Reaktionsprodukt ist zum Ende der Reaktionszeit nicht ganz säurefrei. Deshalb wurde es mit Ca(OH)₂ neutralisiert. Da bei einer Säurezahlbestimmung durch das bei der Bestimmung benötigte Wasser sofortige Hydrolyse des Produktes stattfand und durch diese Reaktion immer mehr Säure frei wurde, war eine Titrationsbestimmung des Säuregehaltes nicht möglich.

Die Säurezahl mußte deshalb theoretisch berechnet werden.

Zur Neutralisation wurde die errechnete Menge an Ca(OH)₂ in das warme Reaktionsprodukt eingetragen und unter Rühren bei 105 °C, Durchleitung von 40 l Luft/h und 500 mbar für 30 Minuten behandelt.

Das neutralisierte Produkt (bei 100 bis 105 °C hochviskos) wird mit Hilfe einer Druckfilternutsche und einem Loeffler-Filter (80 NMO12) bei 100 bis 105 °C unter 3 bar filtriert.

Anschließend wurde das noch heiße Produkt unter ansonsten gleichen Bedingungen über ein Rundfilter (NNG 16 mittelschnell filtrierend) nochmals filtriert.

## Patentansprüche

1. Im menschlichen und tierischen Organismus abbau- und resorbierbare hochfeste Werkstoffe und entsprechende vorgebildete Formkörper, wie Implantate, auf Basis gehärteter (Meth)acrylsäureester von unter Mitverwendung mehrwertiger Alkohole oder mehrwertiger Carbonsäuren (hier mit nachfolgender Umsetzung mit mehrwertigen Alkoholen bzw. deren Derivaten) mehrfunktionell hydroxyl-terminierten Oligomeren von Hydroxycarbonsäuren (mehrfunktionelle (Meth)acrylsäureester) mit bis zu 10 C-Atomen, dadurch gekennzeichnet, daß sie durch Strahlenhärtung und/oder sonstige, jedoch Bor-freie radikalische Polymerisation der mehrfunktionellen (Meth)acrylsäureester dreidimensional vernetzt sind und dabei eine Zugfestigkeit von wenigstens 10 N/mm² aufweisen.

2. Werkstoffe und Implantate nach Anspruch 1, dadurch gekennzeichnet, daß die vernetzten Kunststoffe eine Zugfestigkeit von wenigstens 15 N/mm², vorzugsweise von wenigstens 30 bis 40 N/mm² aufweisen.

3. Werkstoffe und Implantate nach Ansprüchen 1 und 2, dadurch gekennzeichnet, daß sie von Reaktionshilfsmitteln und/oder sonstigen Zusatzstoffen aus der Herstellung (Wirkstoffverunreinigungen) im wesentlichen frei sind, die nach Art und/oder Menge physiologisch unerwünscht sind, wobei hierzu insbesondere im Rahmen ihrer Herstellung eine Abstimmung - jeweils nach Art und Menge - zwischen den Inhibitoren der mehrfunktionellen (Meth)acrylsäureester und den zu ihrer Vernetzung gegebenenfalls eingesetzten Initiatoren bzw. Startersystemen vorgenommen worden ist.

4. Werkstoffe und Implantate nach Ansprüchen 1 bis 3, dadurch gekennzeichnet, daß sie frei sind von physiologisch bedenklichen Lösungsmitteln bzw. herstellungsbedingten Lösungsmittelresten, insbesondere aus der Herstellung der polyfunktionellen (Meth)acrylsäureester oder der Formteil-Ausbildung, und daß ihre Restgehalte an Inhibitoren insbesondere aus der Inhibierung der polyfunktionellen (Meth)acrylsäureester sowie an gegebenenfalls noch vorliegenden Initiatoren aus der Vernetzungsstufe nach Art und Menge physiologisch verträglich gewählt sind.

5. Werkstoffe und Implantate nach Ansprüchen 1 bis 4, dadurch gekennzeichnet, daß sie als Wirkstoffverunreinigung aus der Klasse der Reaktionsinhibitoren Vitamin E und/oder andere körperverträgliche Inhibitoren enthalten.

6. Werkstoffe und Implantate nach Ansprüchen 1 bis 5, dadurch gekennzeichnet, daß sie in den Stufen der Oligomerengewinnung, deren Umwandlung zu mehrfunktionellen (Meth)acrylsäureestern und deren formgestalteter Aushärtung Lösungsmittel-frei hergestellt worden sind.

7. Werkstoffe und Implantate nach Ansprüchen 1 bis 6, dadurch gekennzeichnet, daß sie in einer bevorzugt Initiator-freien gewünschtenfalls mehrstufigen UV-Härtung auf die geforderte Primär-Zugfestigkeit eingestellt worden sind.

8. Werkstoffe und Implantate nach Ansprüchen 1 bis 7, dadurch gekennzeichnet, daß sich ihre Hydroxycarbonsäure-Oligomerbasis aus entsprechenden Verbindungen von Hydroxycarbonsäuren mit 2 bis 6 C-Atomen, insbesondere von Glykolsäure, Milchsäure, Hydroxypropionsäure und/oder Hydroxybuttersäure ableitet.

9. Werkstoffe und Implantate nach Ansprüchen 1 bis 8, dadurch gekennzeichnet, daß sie aus mehrfunktionellen (Meth)acrylsäureestern mit 2 bis 4, vorzugsweise 2 und gegebenenfalls 3 (Meth)acrylsäureresten im Molekül hergestellt worden sind.

10. Werkstoffe und Implantate nach Ansprüchen 1 bis 9, dadurch gekennzeichnet, daß sie auf Oligomeren der Glykolsäure und/oder der Milchsäure basieren, die unter Mitverwendung von Ethylenglykol, 1,2-Propandiol, 1,3-Propandiol und/oder Glycerin zur polyfunktionellen Hydroxyl-Terminierung unter gleichzeitiger Regelung des mittleren Molekulargewichts hergestellt worden sind.

11. Werkstoffe und Implantate nach Ansprüchen 1 bis 10, dadurch gekennzeichnet, daß sie auf Hydroxyl-terminierte Hydroxycarbonsäure-Oligomere eines mittleren Molekulargewichts von etwa 100 bis 600, insbesondere von etwa 200 bis 500 zurückgehen.

12. Werkstoffe und Implantate nach Ansprüchen 1 bis 11, dadurch gekennzeichnet, daß sie einen Gehalt an Füllstoffen vorzugsweise in diskreter Feststoffphase aufweisen, die insbesondere ebenfalls körperresorbierbar sind.

13. Werkstoffe und Implantate nach Ansprüchen 1 bis 12, dadurch gekennzeichnet, daß sie Füllstoffe mit Faserstruktur enthalten, die als Staperfasern, insbesondere aber in Fadenstruktur und/oder als Faden-basiertes Material wie Netze, Gewebe, Gewirke und dergleichen ausgebildet sind.

14. Werkstoffe und Implantate nach Ansprüchen 1 bis 13, dadurch gekennzeichnet, daß sie die Füllstoff-Fasern bzw. Fadenmaterialien in vorgespanntem Zustand in das dreidimensionale Netzwerk eingehärtet enthalten.

15. Werkstoffe und Implantate nach Ansprüchen 1 bis 14, dadurch gekennzeichnet, daß sie Fasern und/oder Fadenmaterial auf Basis Glykolsäure und/oder Milchsäure, insbesondere auf Basis Poly-Milchsäure und/oder auf Polypeptid-Basis enthalten.

16. Werkstoffe und Implantate nach Ansprüchen 1 bis 15, dadurch gekennzeichnet, daß sie Schichtstruktur aufweisen, wobei insbesondere Materialschichten einer vergleichsweise schnelleren Körperresorbierbarkeit mit wenigstens einer Materialschicht verringerter Hydrophilie und/oder erhöhter Hydrolysebeständigkeit abgedeckt sind.

17. Werkstoffe und Implantate nach Ansprüchen 1 bis 16, dadurch gekennzeichnet, daß sie im Kern bzw. in Richtung des Kernes eine oder mehrere Materialschicht(en) erhöhter Abbaugeschwindigkeit aufweisen, die von einer oder mehreren Materialschicht(en) erhöhter Festigkeit gegen Durchtritt der wäßrigen Körperflüssigkeit nach Außen wenigstens anteilsweise abgedeckt sind.

18. Werkstoffe und Implantate nach Ansprüchen 1 bis 17, dadurch gekennzeichnet, daß als Materialschichten erhöhter Festigkeit gegen Wasserdurchtritt Milchsäure-basierte und/oder 1,4-Hydroxybuttersäurebasierte Polymersubstanzen vorliegen.

19. Werkstoffe und Implantate nach Ansprüchen 1 bis 18, dadurch gekennzeichnet, daß sie wenigstens anteilsweise im Formkörper, insbesondere im Formkörperquerschnitt, medizinische Wirkstoffe, z. B. Mittel mit desinfizierender und/oder antibakterieller Wirkung, insbesondere Breitband-Antibiotika, Antiallergika und/oder Mittel zur Stimulierung des Gewebe- bzw. Knochenwachstums enthalten.

20. Werkstoffe und Implantate nach Ansprüchen 1 bis 19, dadurch gekennzeichnet, daß sie in Form von Stäben, Platten, Schienen, Stiften, Schrauben und dergleichen ausgebildet sind.

## Claims

1. High-strength materials which are biodegradable and absorbable in the human and animal body, and corresponding ready-shaped moulded articles, such as implants, based on cured (meth)acrylates of oligomers of hydroxycarboxylic acids having up to 10 carbon atoms, the oligomers being hydroxyl-terminated and polyfunctional due to concomitant use of polyhydric alcohols or of polybasic carboxylic acids (here with subsequent reaction with polyhydric alcohols and/or derivatives of these) (polyfunctional (meth)acrylates), characterized in that they are three-dimensionally cross-linked by radiation curing and/or other free-radical polymerization, but without boron, of the polyfunctional (meth)acrylates, and also have a tensile strength of at least 10 N/mm².

2. Materials and implants according to Claim 1, characterized in that the crosslinked plastics have a tensile strength of at least 15 N/mm², preferably of at least from 30 to 40 N/mm².

3. Materials and implants according to Claims 1 and 2, characterized in that they are substantially free from reaction auxiliaries and/or other additives from their production (active compound contaminants) in any physiologically undesirable amount or form, where to this end there is harmonization in each case of the nature and amount of the inhibitors of the polyfunctional (meth)acrylates and the initiators and/or starter systems which may be used, if desired, for their crosslinking.

4. Materials and implants according to Claims 1 to 3, characterized in that they are free from physiologically hazardous solvents and from solvent residues resulting from their production, in particular the production of the polyfunctional (meth)acrylates or the forming of moulded articles, and that the nature and amount of their residual contents of inhibitors, in particular resulting from the inhibition of the poly-functional (meth)acrylates, and also of the residual contents of any initiators from the crosslinking step which may still be present, are selected so that they are physiologically tolerated.

5. Materials and implants according to Claims 1 to 4, characterized in that they contain, as active compound contaminant from the reaction inhibitors class, vitamin E and/or other inhibitors tolerated by the body.

6. Materials and implants according to Claims 1 to 5, characterized in that their production is solvent-free in the steps of preparing the oligomers, converting them to polyfunctional (meth)acrylates and curing them to give moulded articles.

7. Materials and implants according to Claims 1 to 6, characterized in that their primary tensile strength is established as required in a preferably initiator-free UV curing, which may have more than one step if desired.

8. Materials and implants according to Claims 1 to 7, characterized in that their hydroxycarboxylic acid oligomer basis derives from corresponding compounds of hydroxycarboxylic acids having from 2 to 6 carbon atoms, in particular from glycolic acid, lactic acid, hydroxypropionic acid and/or hydroxybutyric acid.

9. Materials and implants according to Claims 1 to 8, characterized in that they have been produced from polyfunctional (meth)acrylates having from 2 to 4, preferably 2 and if desired 3 (meth)acrylic acid radicals in the molecule.

10. Materials and implants according to Claims 1 to 9, characterized in that they are based on oligomers of glycolic acid and/or of lactic acid, which have been produced with concomitant use of ethylene glycol, 1,2-propanediol, 1.3-propanediol and/or glycerol for poly-functional hydroxyl termination, with, at the same time, control of the mean molecular weight.

11. Materials and implants according to Claims 1 to 10, characterized in that they originate from hydroxyl-terminated hydroxycarboxylic acid oligomers whose mean molecular weight is from about 100 to 600, in particular from about 200 to 500.

12. Materials and implants according to Claims 1 to 11, characterized in that they have a content of fillers, preferably in a discrete solid phase, which are in particular likewise absorbable by the body.

13. Materials and implants according to Claims 1 to 12, characterized in that they contain fillers with fibrous structure, which are in the form of staple fibres, but in particular in the structure of threads and/or as a material based on threads, such as nets, woven fabrics, knitted fabrics and the like.

14. Materials and implants according to Claims 1 to 13, characterized in that they contain the filler fibres and/or filler thread materials in prestressed condition, incorporated into the three-dimensional network during curing.

15. Materials and implants according to Claims 1 to 14, characterized in that they contain fibres and/or thread material based on glycolic acid and/or lactic acid, in particular based on polylactic acid, and/or based on polypeptides.

16. Materials and implants according to Claims 1 to 15, characterized in that they have a layered structure where, in particular, layers of material which have a more rapid absorption by the body compared with others are covered over with at least one layer of material having reduced hydrophilic character and/or increased resistance to hydrolysis.

17. Materials and implants according to Claims 1 to 16, characterized in that in their core and/or towards their core they have one or more layer(s) of material having an increased rate of biodegradation, at least portions of which are covered over towards the outside by one or more layer(s) of material having increased resistance to passage of aqueous body fluid.

18. Materials and implants according to Claims 1 to 17, characterized in that the layers of material having increased resistance to the passage of water are polymeric substances based on lactic acid and/or polymeric substances based on 1,4-hydroxybutyric acid.

19. Materials and implants according to Claims 1 to 18, characterized in that, at least in portions of the moulded article, in particular within the cross section of the moulded article, they contain active medical compounds, e.g. agents with disinfecting and/or antibacterial action, in particular broad-spectrum antibiotics, antiallergics and/or agents to stimulate the growth of tissue and/or of bone.

20. Materials and implants according to Claims 1 to 19, characterized in that they are formed as rods, plates, splints, pins, screws and the like.

## Revendications

1. Matériaux hautement résistants dégradables et résorbables dans l'organisme humain et animal, et articles moulés préformés correspondants, comme des implants, à base d'esters d'acides (méth) acryliques durcis d'oligomères polyfonctionnels à terminaison hydroxyle d'acides hydroxycarboxyliques (esters d'acides (méth)acryliques polyfonctionnels) ayant jusqu'à 10 atomes de carbone, avec utilisation simultanée d'alcools polyvalents ou d'acides carboxyliques polyvalents (ici avec réaction ultérieure avec des alcools polyvalents ou selon les cas leurs dérivés), caractérisés en ce qu'ils sont réticulés de façon tridimensionnelle par un durcissement par irradiation et/ou autre, mais cependant par polymérisation radicalaire sans bore des esters d'acides (méth)acryliques polyfonctionnels, et en ce qu'ils présentent une résistance à la traction d'au moins 10 N/mm2.

2. Matériaux et implants selon la revendication 1, caractérisés en ce que les matières synthétiques réticulées présentent une résistance à la traction d'au moins 15 N/mm2, de préférence d'au moins 30 à 40 N/mm2.

3. Matériaux et implants selon les revendications 1 et 2, caractérisés en ce qu'ils sont essentiellement dépourvus d'adjuvants de réaction et/ou d'autres additifs provenant de la fabrication (impuretés de substances actives), qui selon leur type et/ou leur quantité sont physiologiquement indésirables, où l'on procède à cet effet en particulier dans le cadre de leur fabrication à un ajustement - toujours selon le type et la quantité - entre les inhibiteurs des esters d'acide (méth)acrylique polyfonctionnels et les initiateurs ou systèmes de démarrage le cas échéant utilisés pour leur réticulation.

4. Matériaux et implants selon les revendications 1 à 3, caractérisés en ce qu'ils sont dépourvus de solvants ou de restes de solvants physiologiquement nocifs apparus lors de la fabrication, en particulier dans la préparation des esters polyfonctionnels d'acides (méth)acryliques ou dans la configuration de pièces profilées, et en ce que leur teneur résiduelle en inhibiteurs, provenant en particulier de l'inhibition des esters d'acides (méth)acryliques polyfonctionnels, ainsi qu'en initiateurs éventuellement encore présents provenant de l'étape de réticulation, est choisie pour être physiologiquement compatible en type et en quantité.

5. Matériaux et implants selon les revendications 1 à 4, caractérisés en ce qu'ils contiennent comme impureté de substance active de la classe des inhibiteurs de réaction la vitamine E et/ou d'autres inhibiteurs compatibles avec le corps.

6. Matériaux et implants selon les revendications 1 à 5, caractérisés en ce qu'ils ont été préparés dans Les étapes d'obtention des oligomères, de leur transformation en esters polyfonctionnels d'acide (méth)acrylique et de leur durcissement en une forme moulée sans solvant.

7. Matériaux et implants selon les revendications 1 à 6, caractérisés en ce qu'ils ont été réglés dans un durcissement aux UV, le cas échéant en plusieurs étapes, de préférence sans initiateur, à la résistance primaire à la traction exigée.

8. Matériaux et implants selon les revendications 1 à 7, caractérisés en ce que leur base oligomère d'acide hydroxycarboxylique dérive de composés correspondants d'acides hydroxycarboxyliques présentant de 2 à 6 atomes de carbone, en particulier de l'acide glycolique, de l'acide lactique, de l'acide hydroxypropionique et/ou de l'acide hydroxybutyrique.

9. Matériaux et implants selon les revendications 1 à 8, caractérisés en ce qu'ils sont fabriqués à partir d'esters d'acides (méth)acryliques polyfonctionnels comportant de 2 à 4, de préférence 2 et le cas échéant 3 esters d'acide (méth)acrylique dans la molécule.

10. Matériaux et implants selon les revendications 1 à 9, caractérisés en ce qu'ils sont à base d'oligomères de l'acide glycolique et/ou de l'acide lactique, qui sont préparés avec utilisation simultanée d'éthylèneglycol, de 1,2-propanediol, de 1,3-propanediol et/ou de glycérine pour la terminaison hydroxyle polyfonctionnelle avec réglage simultané du poids moléculaire moyen.

11. Matériaux et implants selon les revendications 1 à 10, caractérisés en ce qu'ils reposent sur des oligomères d'acides hydroxycarboxyliques à terminaison hydroxyle d'un poids moléculaire moyen d'environ 100 à 600, en particulier d'environ 200 à 500.

12. Matériaux et implants selon les revendications 1 à 11, caractérisés en ce qu'ils présentent une teneur en charges de préférence en phase solide discrète, et qui est en particulier également résorbable par le corps.

13. Matériaux et implants selon les revendications 1 à 12, caractérisés en ce qu'ils contiennent des charges à structure de fibres, qui sont formées en fibres coupées, mais en particulier en une structure de fil et/ou sous forme de matériau à base de fils comme les filets, les tissus, articles de textile tissés, etc.

14. Matériaux et implants selon les revendications 1 à 13, caractérisés en ce qu'ils contiennent des fibres de charges ou selon les cas des matériaux en fils à l'état pré-tendu, durcis dans le réseau tridimensionnel.

15. Matériaux et implants selon les revendications 1 à 14, caractérisés en ce qu'ils contiennent des fibres et/ou des matériaux en fils à base d'acide glycolique et/ou d'acide lactique, en particulier à base d'acide polylactique et/ou à base de polypeptide.

16. Matériaux et implants selon les revendications 1 à 15, caractérisés en ce qu'ils présentent une structure en couche, dans laquelle en particulier des couches de matériau ayant une résorbabilité corporelle relativement rapide sont recouvertes avec au moins une couche de matériau d'hydrophilie abaissée et/ou de résistance accrue à l'hydrolyse.

17. Matériaux et implants selon les revendications 1 à 16, caractérisés en ce qu'ils présentent dans le noyau ou dans la direction du noyau une ou plusieurs couches de matériau de vitesse de dégradation accrue, qui sont recouvertes vers l'extérieur au moins en partie d'une ou plusieurs couches de matériau de résistance accrue à la pénétration des liquides corporels aqueux.

18. Matériaux et implants selon les revendications 1 à 17, caractérisés en ce qu'ils ont comme couche de matériau de résistance accrue à la pénétration de l'eau des substances polymères à base d'acide lactique et/ou d'acide 1,4-hydroxybutyrique.

19. Matériaux et implants selon les revendications 1 à 18, caractérisés en ce qu'ils contiennent au moins en partie dans des corps moulés, en particulier dans une coupe de corps moulé, des substances médicales actives, par exemple des agents à action désinfectante et/ou anti-bactérienne, en particulier des antibiotiques à large spectre, des anti-allergiques et/ou des agents pour stimuler la croissance des tissus ou selon les cas des os.

20. Matériaux et implants selon les revendications 1 à 19, caractérisés en ce qu'ils sont en forme de bâtonnets, de plaques, de gouttières, de stylets, de vis, et similaires.
